# EUROPEAN PATENT APPLICATION

(11) **EP 1 542 195 A1**
(43) Date of publication of application: **15.06.2005**
(21) Application number: 03797529.9
(22) Date of filing: 27.08.2003
(51) Int. Cl.: G09F 3/02, G09F 13/42, G09F 19/12, G06K 19/00, B42D 15/10

(54) **PRODUCT INFORMATION DISPLAY BODY AND PRODUCT AUTHENTICATION METHOD**

(30) Priority: 27.08.2002 JP 2002246658
(71) Applicant: FP Corporation, Fukuyama-shi Hiroshima 721-0952 (JP); Plagenom Co., Ltd., Osaka-shi, Osaka 542-0086 (JP); Fukui, Shinya, Toyonaka-shi, Osaka 560-0002 (JP)
(72) Inventor: FUKUI, Shinya, Toyonaka-shi, Osaka 560-0002 (JP); SATO, Morimasa, Minato-ku, Tokyo 106-0047 (JP); TSUBONE, Masahiro, Koga-shi, Ibaraki 306-0002 (JP); MAEDA, Kazunori, Yao-shi, Osaka 581-0832 (JP)
(74) Representative: Viering, Jentschura & Partner
(86) International application number: PCT/JP2003/010827
(87) International publication number: WO 2004/027738

(57) **Abstract**

There is provided an economical product information display body A capable of increasing credibility of the product information and sufficiently assuring product reliability and safety at a low cost. The display body A is attached to a product and has a visible information display C relating to the product and invisible information identification means D. The invisible information identification means D is composed of an information presenting substance consisting of one or more elements emitting fluorescence when radiated by an electromagnetic wave of a particular wavelength range or a compound consisting of two or more elements or a substance including these elements or a compound. One or more information of the product information display by the visible information display is correlated to the information presenting substance.

## Description

### Field of the Invention

The present invention relates to a product information display body, such as a label, a packaging bag, a packaging box, a packaging band, or a tag, attached to a product, such as fresh food, processed food, or industrial good, as a visible information display about the product.

### Description of the Background Art

A variety of products including fresh foods, processed foods, and industrial goods are attached with display bodies, such as labels, packaging bags, packaging boxes, packaging bands, or tags, for displaying details of information about the products including producers, producing dates, and producing locations (referred to as product information hereinafter). This allows consumers, retailers, or wholesalers to know from the information on the display body when, where, and by whom the products were produced.

However, there have been a lot of false conducts carried out at the steps of production, distribution, and sale such as attachment of a display body carrying false product information at the producing location or replacement of an authentic display body with another display body carrying the false product information. As a result, the credibility of the information about the product will be declined thus failing to guarantee the reliability and safety of the product.

It is then contemplated for improving the credibility of the product information to have a display body accompanied with invisible information identification means where one or more pieces of the product information are recorded. Such invisible information identification means may be a magnetic recording chip for magnetically recording the information or an IC chip for casting the information. Accordingly, the product information is read out from the invisible information identification means and compared with the information visibly exhibited on the display body for authenticating the product information.

However, the product information recorded in magnetic recording chips or IC chips will easily be read and analyzed or, if worse, replaced with false information, hence permitting its modifications or forgeries with no difficulty. As the credibility of the product information remains not high, the reliability and safety of the product can hardly be ensured. Also, the magnetic chips or IC chips are still expensive in the price and will have little opportunity to be carried on low priced products such as fresh foods.

The present invention has been developed in view of the above aspects and its object is to provide a product information display body which can improve the credibility of the product information thus to ensure the reliability and safety of the product while being low in the cost and a product authentication method for judging whether the product identified with the product information display body is authentic or not.

### SUMMARY OF THE INVENTION

A product information display body according to the present invention is assigned with pieces of information about a product and attached as a visible information display to the product. The product information display body is characterized by that the invisible information identification means is provided in the display body, wherein the invisible information identification means comprises an information presenting substance which is one or more elements emitting fluorescence when exposed to a specific wavelength range of electromagnetic wave, a compound composed of two or more of the elements, or a substance including the element or the compound, and the information presenting substance is correlated with one or more pieces of the information about the product displayed on the visible information display.

The term "product" means any of all products including fresh foods, processed foods, and other industrial products. The term "information about a product" means each piece of product information including the name of a producer, the date of production, the location of production, the location of sale, the quality of products, the materials to be used, the effect or advantage of products, the application, the quantity, the shape, the price, the data about agricultural chemicals (e.g., time of application, quantity, and type), the processing conditions (temperature and duration), the period of tasting, the period of holding, the ingredients, the additives, the invoice or inventory data of distributors, the history of delivery by a forwarder, the materials, processing steps, and lot number by a processor, and the invoice and display data of a seller. The term "display body" means a product information carrier including labels, packaging bags, packaging boxes, packaging bands, and tags.

In action, when the display body is exposed to a specific range of wave, its information presenting substance emits fluorescence which is then measured and analyzed to detect the product information assigned to the information presenting substance. This hardly permits any third party to access the product information with the use of the invisible information identification means. Also, even if the third party has a skill for detecting the fluorescence emitted from the information presenting substance, it may substantially fail to produce a false version of the display body because the production of the display body including the information representing substance which can emit the same fluorescence requires special techniques and facilities.

Since the product information lodged in the invisible information identification means is inhibited from being illegally duplicated or modified, it can support the credibility of the visible information display. As a result, the credibility of the product information can be enhanced thus ensuring the reliability and safety of the product.

Also, the display body accompanied with the invisible information identification means of the information representing substance is economical as significantly lower in the production cost than any conventional invisible information identification means such as a magnetic storage or an IC chip. Accordingly, the display body can be applied to low cost products including fresh foods. Such fresh foods tagged with the display body can thus be improved in the market reliability among consumers and distributors and their safety level will be enhanced.

Preferably, the information presenting substance is arranged to emit one or more line spectrum when exposed to the specific wavelength range of electromagnetic wave.

As the line spectrum from the information presenting substance is very narrow in the wavelength width and high in the intensity, it can be measured at higher accuracy.

Preferably, the information presenting substance contains a transition element of incomplete 3d shell and/or a transition element of incomplete 4f shell.

As a result, the information presenting substance assuredly emits the aforementioned line spectrum, thus contributing to accurate measurement of the fluorescence of the information presenting substance.

Also, a product authentication method according to the present invention is provided for examining the authentication of a product using a product information display body which is assigned with pieces of information about a product as a visible information display and is provided with an invisible information identification means composed of an information presenting substance correlated with one or more pieces of the information about the product displayed on the visible information display. The product authentication method comprises a step of exposing the product information display body to a specific wavelength range of electromagnetic wave and detecting fluorescence emitted from the information presenting substance of the product information display body with the use of detecting means, a step of identifying the information about the product correlated with the information presenting substance based on a measurement of the fluorescence emitted from the information presenting substance, and a step of comparing the information about the product with the pieces of information provided as the visible information display on the product information display body to judge whether the product attached with the product information display body is authentic or not.

Accordingly, the products provided with the product information display body can be judged simply and certainly. When the products have been judged by a retailer that they are authentic, they can be received from the wholesaler at a level of security and their supply from the retailer to the consumers can be made with a degree of insurance. This also allows every consumer to select and purchase the products from the retailer at soundness. Accordingly, the distribution of each product will highly be encouraged.

Moreover, the product authentication method may preferably be modified in which both the information about the product correlated with the information presenting substance and the measurement of the fluorescence emitted from the information presenting substance are consolidated by a data bank which is positioned distanced from a location of measuring the fluorescence emitted from the information presenting substance.

As a result, the product information in the invisible information identification means consolidated by the data bank will be improved in the credibility and hardly be obtained and decoded by a third party.

### BRIEF DESCRIPTION OF THE DRAWINGS

Fig. 1A is a perspective view showing a product information display body attached to a product, where the product information such as a producer, a date of production, or a location of production, is labeled on a box of cucumbers in the form of the visible information display as well as an invisible information display;
Fig. 1B is a perspective view showing the product information display body attached to a product, where the product information such as a producer, a date of production, or a location of production, is labeled on a bag of spinach in the form of the visible information display as well as an invisible information display;
Fig. 1C is a perspective view showing the product information display body attached to a product, where the product information such as a producer, a date of production, or a location of production, is labeled on a head of cabbage in the form of the visible information display as well as an invisible information display;
Fig. 2 is a spectrum analysis diagram showing an example of the measurement of fluorescence emitted from an information presenting substance of the display body;
Fig. 3A illustrates a table of the relationship between the product information and the information presenting substance, where a variety of products are assigned with different types of the information presenting substance respectively;
Fig. 3B illustrates a table of the relationship between the product information and the information presenting substance, where a variety of producers are assigned with different contained amounts of the information presenting substance respectively;
Fig. 3C illustrates a table of the relationship between the product information and the information presenting substance, where digits of a numeral representing the product are assigned with different types of the information presenting substance and different contained amounts of the information presenting substance respectively;
Fig. 3D is a profile showing the relationship between the product information and the product presenting substance, where the intensity of visual spectral line emitted from the information presenting substance (a rare-earth elements contained compound) assigned to each producer is decayed with time in a spectrum of visible light;
Fig. 4 is a schematic view showing the entire arrangement of a product distributing system;
Fig. 5 is a block diagram showing a hardware arrangement of a reader apparatus shown in Fig. 4; and
Fig. 6 is a flowchart of steps in the product distributing system of Fig. 4.

### DESCRIPTION OF THE PREFERRED EMBODIMENTS

Some embodiments of the present invention will be described referring to the relevant drawings.

### (Product information display body)

A product information display body (A) of the embodiment which may be a label, a packaging bag, a packaging box, a packaging band, or a tag attached to a product (B) carries the visible information display (C) for exhibiting pieces of information about the product (B) (referred to as product information hereinafter) and is provided with the invisible information identification means (D).

The product (B) may be a fresh food such as agricultural product, meat, or fish, a processed food such as canned, frozen, or retorted food, an industrial product such as a household electric appliance, a decoration, an ornament, a pair of shoes, a bag, an accessory, a watch, a ring, a garment, a stationary, a kitchenware, or an interior good, or any other market available product.

The product information may be any types of information about the product (B). For example, the product information includes the producer, the producing date, the producing location, the selling location, the quality, the materials, the benefits, the applications, the quantity, the shape, the price, the pesticide used (date of application, quantity, and type), the processing conditions (temperature and time), the tasting period, the quality preservation period, the additives and the ingredients of the product (B). The product information may also include any extra data provided during the distribution of the product (B), the invoice or inventory data of distributors, the history of delivery by a forwarder, the materials, processing steps, and lot number by a processor, and the invoice and display data of a seller.

The product information is substantially provided as the visible information display (C) on the display body (A). More particularly, the product information in the visible information display (C) may be printed down in characters, symbols, or bar codes on the display body (A).

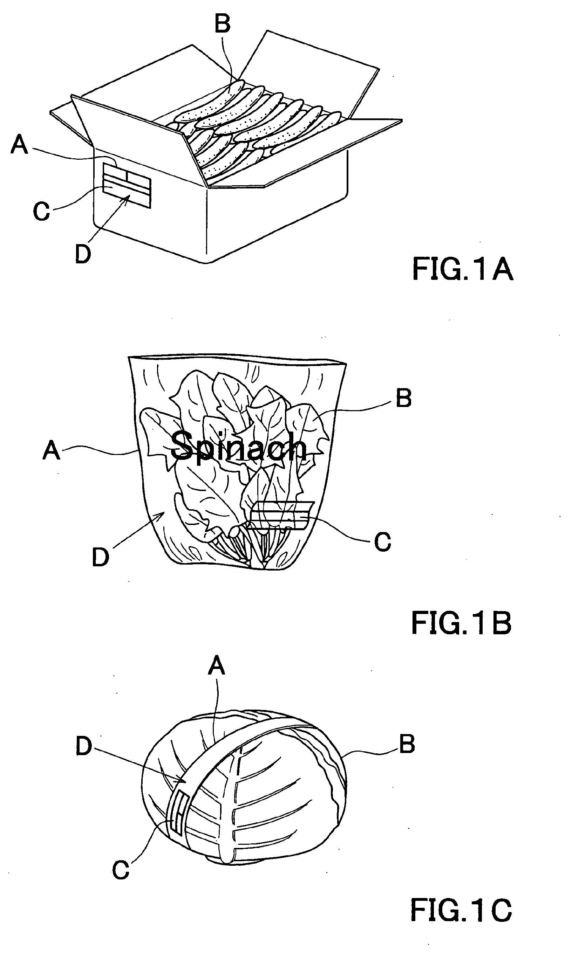

For example, Fig. 1A illustrates the display body or label (A) carrying the visible information display (C) such as the name of a producer, the date of production, and the location of production of a case of cucumbers (B) as the product. Fig. 1B illustrates the display body or packaging bag (A) carrying the visible information display (C) such as the name of a producer, the data of production, and the location of production of a bundle of spinach (A). Fig. 1C illustrates the display body or packaging band (A) carrying the visible information display (C) such as the name of a producer, the data of production, and the location of production of a head of cabbage (B).

The invisible information identification means (D) comprises an information presenting substance which is one or more elements emitting fluorescence upon being exposed to a specific wavelength range of electromagnetic wave, a compound of two or more of such elements, or a substance including such elements or compound. The information presenting substance of the invisible information identification means (D) is assigned with one or more pieces of the product information of the visible information display (C).

In case that its emitting florescence is X ray, the information presenting substance is preferably one or more elements, a compound of two or more elements (e. g. , oxide, sulfide, or organic complex), or a substance including such elements or compound which is not generally contained in the display body (A).

The one or more elements not generally contained in the display body (A) may preferably be selected from a series of elements at the atomic number from 31 to 88 or namely lanthanide or more particularly neodymium (Nd), samarium (Sm), europium (Eu), gadolinium (Gd), terbium (Tb), Holmium (Ho), and their combination. Those preferred elements are rarely found in the commonly available materials such as plastic, paint, ink, paper, fiber, and metal and their spectrum can easily be measured and analyzed. Also, those elements are inexpensive, sanitary, and commercially available in the form of oxides.

When its line spectrum has a narrow wavelength range in the spectrum ranging from infrared light to ultraviolet light, the information presenting substance may consist of one or two elements, a compound of two or more of such elements (e.g., oxide, sulfide, or organic complex), or their combination.

The element or compound which emits a line spectrum with a narrow wavelength spectrum is selected from crystal provided with a transition element of incomplete 3d shell and/or a transition element of incomplete 4f shell, glass provided with a transition element of incomplete 3d shell and/or a transition element of incomplete 4f shell, and complex based on a transition element with an incomplete 3d shell and/or a transition element of incomplete 4f shell. When exposed to a given wavelength range of electromagnetic wave or preferably a range of electromagnetic wave from infrared light to ultraviolet light or more preferably a range of electromagnetic wave from visible light to far infrared light, the information presenting substance emits a line spectrum with a narrow wavelength and a higher level of the intensity of fluorescence which corresponds to the range of the electromagnetic wave and can thus be measured at higher accuracy.

Also, the information presenting substance may comprise a base material provided with a preferred transition element or compound such as yttrium oxide (Y₂O₃) containing europium ions (Eu³⁺). The base material is preferably selected from oxides, sulfides, nitrides, hydroxides, halides, mixed crystals, and amorphous or glass materials. For example, the transition element may be contained in a chemically coupling form such as chelete compound, substituted in its crystalline grating with other atoms or ions, inserted in a crystalline grating, or embedded in voids in glass.

The information presenting substance may also be coated with a surface decorative agent, such as heavy hydrogen or organic, or covered with any other material than the base material. This permits the information presenting substance to be fixed in the particle form or the structure, thus improving the efficiency of the emission of fluorescence. Also, the information presenting substance can easily be dissolved in a specific solvent and enhanced in the affinity to neighbor materials.

The information presenting substance may be applied to a region at the inner or outer side of the material (A) by any appropriate manner such as blowing, spraying, coating, absorption, injection, filling, attachment, impregnation, mixture, doping, or chemical coupling (polymerization, bridging, or ion bonding).

More specifically, when the material (A) is made of a plastic resin material, it may be shaped directly after dry blended by a drum tumbler, compounded by an extruder, or milled by an internal mixer or heating rolls. Alternatively, the process may be carried out after the material (A) is subjected to master batch processing. Alternatively, the material (A) may be impregnated with the information presenting substance with the use of a lubricant, such as fatty amide, fatty metallic salt, or fatty ester, for ensuring uniform distribution and dispersion of the substance.

The information presenting material may be applied directly to the display body (A) or mixed with ink or paint used for the visible information display (C) prior to printing the visible information display (C) on the display body (A).

The amount of the information presenting substance to be applied to the material (A) is controllably determined to such a desired level that the material (A) remains unchanged in the pertinent characteristics in order to give a minimum effect on the external appearance or physical properties.

Although the desired amount of the information presenting substance for not largely affecting the pertinent characteristics of the material (A) is variable depending on the type of the material (A), it preferably ranges from 0.1 ppm to 1000 ppm (including 0.1 ppm and 1000 ppm) or more preferably from 0.5 ppm to 200 ppm (including 0.5 ppm and 200 ppm).

It is noted that the amount is not smaller than 0.1 ppm in view of the accuracy of measurement which is commonly employed in the relevant field. As the amount is not greater than 1000 ppm, it can hardly affect the external appearance or physical properties of the material (A). Also, the preferable range from 0.5 ppm to 200 ppm is determined for ensuring the reliability of measurement while minimizing the cost of the production. Also, the material (A) can further be protected from declining its pertinent characteristics.

The information presenting substance is assigned with one or more pieces of the product information which are written on the visible information display (C). More specifically, the type of the information presenting substance, the contained amount, the characteristic of decay with time, the history of production, and/or their combination and one or more pieces of the product information displayed by the visible information display (C) correspond with each other, and since such information presenting substance is included in the display body (A), one or more pieces of the product information are contained in the display body (A) invisibly.

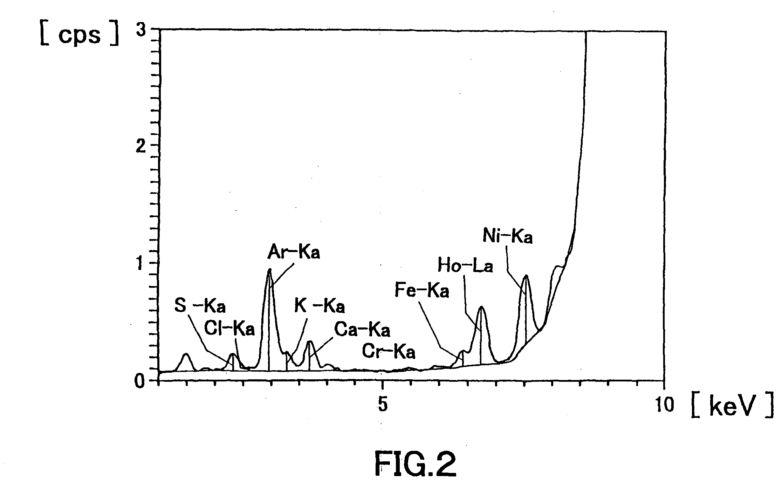

When the display body (A) is exposed to a specific wavelength range of electromagnetic wave, its information presenting substance emits fluorescence which is then measured and analyzed to detect the type, the contained amount, the characteristic of decay with time, the history of production, and/or their combination of the information presenting substance and thus identify the product information. The result of measurement of the fluorescence can be expressed in the form of a spectrum diagram, such as shown in Fig. 2, illustrating the energy level (indicating the type of the information presenting substance) along the horizontal axis and the intensity of the fluorescence (indicating the amount of the information presenting substance) along the vertical axis.

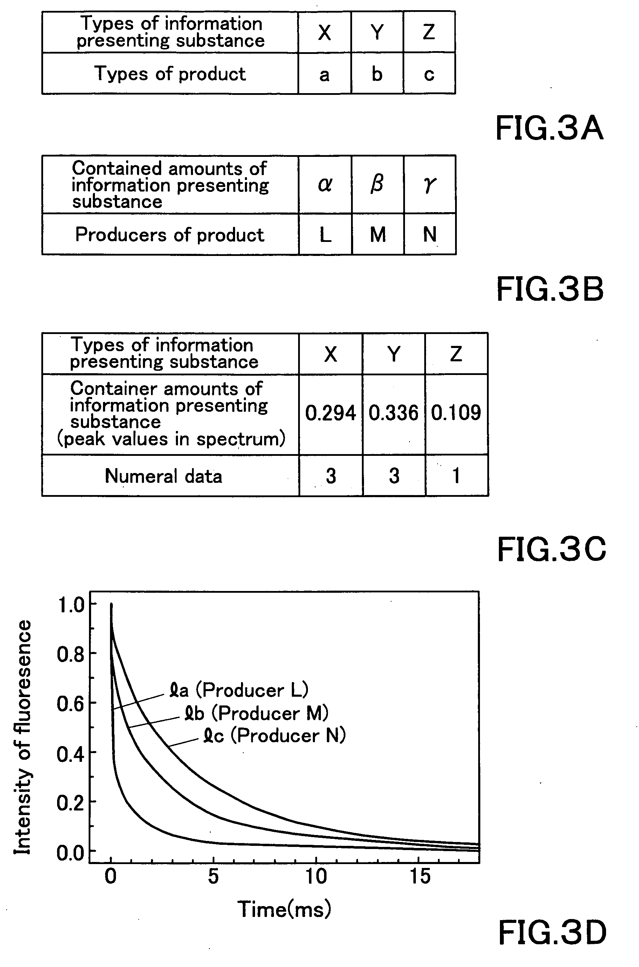

Fig. 3A illustrates an example of the product information where three types (a, b, and c) of the product (B) are assigned with three types (X, Y, and Z) of the information representing substance respectively. Accordingly, the type (a, b, or c) of the product (B) can be identified from the measurement of the fluorescence which indicates the type (X, Y, or Z) of the information presenting substance in the display body (A).

Fig. 3B illustrates another example of the product information where three produces (L, M, and N) of the product (B) are assigned with three different amounts (α, β, and γ) of the information representing substance respectively. Accordingly, the producer (L, M, or N) of the product (B) can be identified from the measurement of the fluorescence which indicates the amount (α, β, or γ) of the information presenting substance in the display body (A).

Fig. 3C illustrates a further example of the product information as a numeral data of the product (B) where three digits of the numeral data are assigned with three types (X, Y, and Z) of the information representing substance respectively and the value of each digit represents the amount of the information presenting substance. Accordingly, the numeral data of the product (B) can be identified from the measurement of the fluorescence which indicates the type (X, Y, or Z) of the information presenting substance and its amount in the display body (A). The numeral data can hence replace a bar code and allows a variety of the product information to be applied to the display body (A) through using different types X, Y, and Z of the information representing substance. Each digit of the numeral data in the example shown in Fig. 3C is determined by multiplying the amount (at peak) of the information representing substance ten times and rounding its multiplication.

Fig. 3D illustrates a still further example of the product information where the three producers (L, M, and N) of the product (B) are assigned with three characteristics of attenuating with time (1a, 1b, and 1c) of the information representing substance respectively. Accordingly, the producer (L, M, orN) of the product (B) can be identified from the measurement of the fluorescence which indicates the characteristic of decay with time (1a, 1b, or 1c) of the information presenting substance in the display body (A). The characteristic of decay with time is decay in the intensity of the fluorescence, at the spectrum of visible light, which has been emitted from the information representing substance (a rare-earth element contained compound). In Fig. 3D, the characteristic of decay with time at the spectrum of visible light represents three different types of the information representing substance.

When the display body (A) is exposed to a specific range of wave, its information presenting substance emits fluorescence which is then measured and analyzed to detect the product information assigned to the information presenting substance. This hardly permits any third party to access the product information with the use of the invisible information identification means. Also, even if the third party has a skill for detecting the fluorescence emitted from the information presenting substance, it may substantially fail to produce a false version of the display body because the production of the display body including the information representing substance which can emit the same fluorescence requires special techniques and facilities.

The information assigned to the information presenting substance may not directly be written on but related to the visible information display (C) for ensuring the credibility of the visible information display (C).

Since the product information lodged in the invisible information identification means (D) is inhibited from being illegally duplicated or modified, it can support the credibility of the invisible information display (D). As a result, the credibility of the product information can be enhanced thus ensuring the reliability and safety of the product.

Also, the display body accompanied with the invisible information identification means (D) of the information representing substance is economical as significantly lower in the production cost than any conventional invisible information identification means such as a magnetic storage or an IC chip. Accordingly, the display body of this embodiment can be applied to low cost products including fresh foods. Such fresh foods tagged with the display body can thus be improved in the market reliability among consumers and distributors and their safety level will be enhanced.

### (Product distributing system)

A product distributing system using the product information display (A) will now be described.

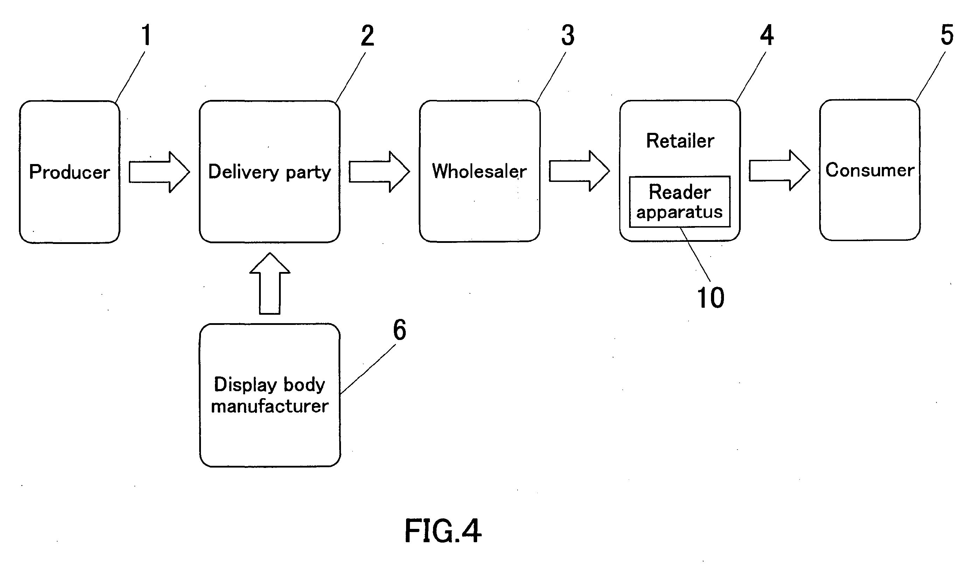

Fig. 4 is a schematic view showing an entire arrangement of the product distributing system.

Shown in Fig. 4 are a producer 1 for producing products (B), a delivery party (or person) 2 for packaging the products (B) received in bulk from the producer 1, a wholesaler 3 for buying the products (B) from the delivery party 2 through bidding at a wholesale market, a retailer 4 such as a supermarket store for receiving the products (B) from the wholesaler 3, a consumer 5 for purchasing the products (B) from the retailer 4, and a display body manufacturer 6 for manufacturing the display body (A). There is also a reader apparatus 10 provided at the shop or warehouse of the retailer 4 for reading the product information saved invisibly in the display body (A).

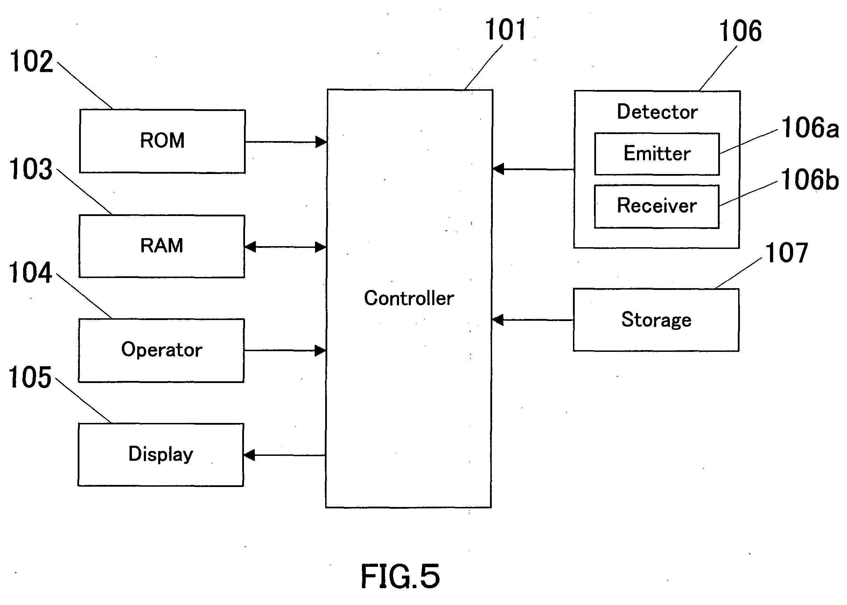

The reader apparatus 10 comprises, as shown in Fig. 5, a controller 101 provided with a central processing unit (CPU) for controlling the action of each component, the display of display screens, the input and output of data, and a variety of arithmetic operations, a ROM (read only memory) 102 for storage of programs for carrying out each step of processing action, a RAM (random access memory) 103 for temporal storage of the data and programs for the processing action, an operator 104 including an array of control button switches, a display 105 for displaying the display screens, a detector 106 for detecting of fluorescence emitted from the information presenting substance of the display body (A), and a storage 107 for storage of a reference table indicating the relationship between the product information and the information presenting substance.

The detector 106 consists mainly of an emitter 106a and a receiver 106b. A specific wavelength range of electromagnetic wave is emitted from the emitter 106a and fallen on the display body (A). When exposed to the electromagnetic wave, the information presenting substance of the display body (A) emits fluorescence which is then received and examined by the receiver 106b of the detector 106.

The storage 107 is arranged in which a reference table indicating the relationship between the product information and the information presenting substance is stored. As shown in Figs. 3A to 3D, the reference table has each piece of the product information such as a producer of the product (B), a date of the production, or a location of the production linked with a characteristic data of the information presenting substance such as a contained amount, a spectrum data, a decay with time, or a history of the production.

The controller 101 has a function of identifying the information presenting substance from the measurement of the fluorescence received by the detector 106, comparing the information presenting substance with the product information listed in the reference table read from the storage 107, and displaying the product information on the display 105. The controller 101 may also have a function of, if the product information fails to be extracted due to, e. g. , lack of the information presenting substance in the display body (A), displaying the lack of the information presenting substance on the display 105.

### (Procedure in product distributing system)

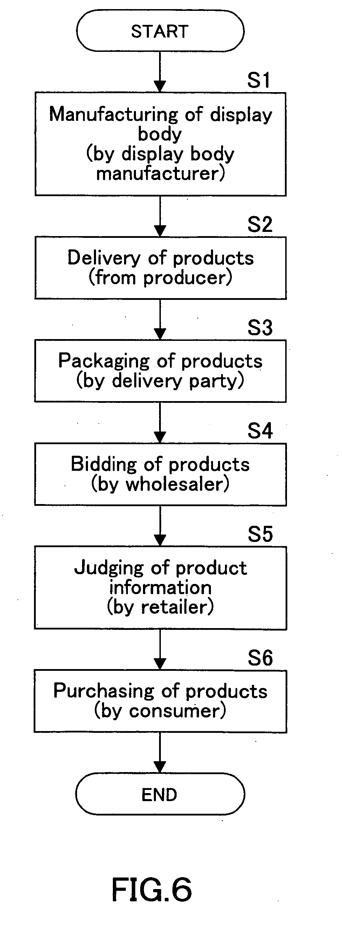

A procedure of actions in the product distributing system will be described referring a flowchart shown in Fig. 6. Each step of the procedure will be abbreviated to simply S throughout the description and the drawings.

The procedure starts with a display body manufacturer 6 manufacturing the display body (A) accompanied with the visible information display (C) which carries the product information including a producer of the products (B), a date of the production, and a location of the production (S1). In particular, the information presenting substance assigned with one or more of the product information is used for manufacturing the display body (A), hence allowing one or more of the product information to be lodged invisibly in the display body (A).

This is followed by transferring the products (B) from a producer 1 to the delivery party 2 (S2).

Then, the products (B) received from the producer 1 are packaged and tagged with the display body (A) received from the display body manufacturer 6 by the delivery party 2 before delivered to a wholesale market (S3).

The products (B) are bid and purchased by a wholesaler 3 at the wholesale market and then dispatched to a retailer 4 such as a supermarket store (S4).

The products (B) received from the wholesaler 3 are then examined by the retailer 4 to read the invisible product information from the display body (A) using the reader apparatus 10. Simultaneously, the product information clearly written on the visible information display (C) attached to the display body (A) is visibly read out. It is then examined from the two readings of the product information whether the products (B) are authentic or not (S5).

The products (B) judged authentic are then purchased by consumers 5 (S6).

As the products (B) are judged simply and certainly by the retailer 4 whether they are authentic or not, they can be transferred from the wholesaler to the retailer at a level of security and their supply from the retailer to the consumers can be made with a degree of insurance. This also allows every consumer 5 to select and purchase the products (B) from the retailer at soundness. Accordingly, the distribution of each product will highly be encouraged.

Although the product distributing system of this embodiment permits the retailer 4 to judge the authentication of the product information, it may be modified in that the product information is examined by the delivery party 2, the wholesaler 3 , or the consumer 5. When the product information is examined by the consumer 5, the reader apparatus 10 can preferably be sized compact allowing the consumer 5 to expose the display body (A) to a specific wavelength range of electromagnetic wave and read the product information from of fluorescence emitted from the information presenting substance of the display body (A).

In the product distributing system of this embodiment, the reader apparatus 10 is provided with the reference table and used by the retailer 4 for reading an invisible version of the product information from the display body (A). Alternatively, the reading and judging of the invisible product information may be carried out separately by one of reader peripherals provided at different sites for detecting the information presenting substance or its fluorescent spectrum information and a data bank or server computer provided far from the sites and connected over a network to the reader peripherals. In the latter case, the data bank examines the information presenting substance or the fluorescent spectrum information received from the reader peripheral from the reference table which indicates the relationship between the information presenting substance or the fluorescent spectrum information and the product information. As the product information assigned with the information presenting substance or the fluorescent spectrum information is authenticated by the data back, it can be fed back to the reader peripheral. In response, the reader peripheral displays the product information fed back from the data bank. Accordingly, two or more of the product information can exclusively be controlled by the data bank.

Although the product distributing system of this embodiment is accompanied with, but not limited to, the delivery party 2, the wholesaler 3, and the retailer 4, it may include a local dealer, an associate dealer, a trading company, or any other distributor.

Although the information presenting substance of the display body (A) is assigned with two or more pieces of the production information visibly provided on the visible information display (C), it may carry any other information. Any other information not legible on the display body (A) can thus be lodged invisibly in the display body (A).

Alternatively, the relationship between the information presenting substance and the production information may be updated at equal intervals of a time. This permits any third party to illegally duplicate or modify the product information with much difficulty. Accordingly, the credibility of the product information can further be improved.

Although the product (B) is attached with a single display body (A) in the embodiment, it may carry two or more of the display bodies (A). For example, a bundle of spinach (B) shown in Fig. 2B may be attached with a label and packaged in a bag which indicate different items of the product information from each other. More specifically, the bundle of spinach (B) is packaged in the bag (A) which carries visibly and invisibly a fixed item of the product information such as a name of the product, "Spinach", while a label which carries visibly and invisibly variable items of the product information including the date of production, the date of consumption, the tasting period, and the holding period is glued to the packaging bag (A). This allows the label carrying variable items of the product information to be prepared separately of the packaging bag (A) which carried a fixed item of the product information. Accordingly, the variable items of the product information which are varied from time to time can be attached to the product (B) at the best of timing.

Although the display body (A) in the product distributing system is provided with the product information determined at the delivery step, it may be loaded later with any other extra information of the visible information display (C) which is determined or added by an associate dealer or distributor at each intermediate step of the distribution of the product (B). Such extra information determined or added at each intermediate step of the distribution includes, for example, the invoice or inventory data of distributors, the history of delivery by a forwarder, the materials, processing steps, and lot number by a processor, and the invoice and display data of a seller. The method of providing the display body (A) with any extra of the visible information display (C) may involve printing of characters, symbols, or bar codes of the product information.

Alternatively, any other extra information determined or added at each intermediate step of the distribution of the product (B) may be applied to the display body (A) in the form of the invisible information identification means (D). The method of applying the invisible information identification means (D) to the display body (A) may involve coating the display body (A) with a type of ink containing the information presenting substance assigned with the extra information, printing a pattern of such ink, or bonding a seal or label made from the information presenting substance to the display body (A).

Any extra product information determined or added at each step of the distribution of the product (B) may be applied to the display body (A) through newly assigning with the existing information presenting substance of the display body (A). In this case, the information presenting substance and its assigned product information are preferably combined to a system which is controlled by a data bank or server computer. In action, the extra product information is transmitted from its distributor to the data bank where it is technically assigned to the existing information presenting substance of the display body (A).

## Claims

1. A product information display body assigned with pieces of information about a product and attached as a visible information display to the product, **characterized by** that:
invisible information identification means is provided in the display body, wherein
the invisible information identification means comprises an information presenting substance which is one or more elements emitting fluorescence when exposed to a specific wavelength range of electromagnetic wave, a compound composed of two or more of the elements, or a substance including the element or the compound, and
the information presenting substance is correlated with one or more pieces of the information about the product displayed on the visible information display.

2. The product information display body according to claim 1, wherein the information presenting substance is arranged to emit one ormore line spectrum when exposed to the specific wavelength range of electromagnetic wave.

3. The product information display body according to claim 1 or 2, wherein the information presenting substance contains a transition element of incomplete 3d shell and/or a transition element of incomplete 4f shell.

4. A product authentication method for examining the authentication of a product using a product information display body which is assigned with pieces of information about a product as a visible information display and is provided with an invisible information identification means composed of an information presenting substance correlated with one or more pieces of the information about the product displayed on the visible information display, comprising the steps of:
exposing the product information display body to a specific wavelength range of electromagnetic wave and detecting fluorescence emitted from the information presenting substance of the product information display body with the use of detecting means;
identifying the information about the product correlated with the information presenting substance based on a measurement of the fluorescence emitted from the information presenting substance; and
comparing the identified information about the product with the pieces of information provided as the visible information display on the product information display body to judge whether the product attached with the product information display body is authentic or not.

5. The product authentication method according to claim 4, wherein both the information about the product correlated with the information presenting substance and the measurement of the fluorescence emitted from the information presenting substance are consolidated by a data bank which is positioned distanced from a location of measuring the fluorescence emitted from the information presenting substance.
